# FASCICULE DE BREVET EUROPEEN

(11) **EP 0 984 010 B1**
(45) Date de publication et mention de la délivrance du brevet: **20.06.2001**
(21) Numéro de dépôt: 99402112.9
(22) Date de dépôt: 25.08.1999
(51) Int. Cl.: C07D 401/04, A61K 7/13, C07D 403/04, C07D 215/24

(54) **Utilisation en teinture capillaire de condensats de quinoline-5,8-diones ou de quinoxaline-5,8-diones et de pyrroles, anilines ou indoles substitués.**
Verwendung in Haarfarbstoffen von Chinolin-5,8-dionen oder Chinoxalin-5,8-dionen kondensiert mit substituierten Pyrrolen, Anilinen oder Indolen
Use in hair dyes of quinolin-5,8-diones or quinoxalin-5,8-diones condensed with substituted pyrroles, anilines or indoles

(30) Priorité: 01.09.1998 FR 9810919
(43) Date de publication de la demande: 08.03.2000
(73) Titulaire: L'OREAL, 75008 Paris (FR)
(72) Inventeur: Baudry, Richard, 75013 Paris (FR); Maignan, Jean, 93290 Tremblay en France (FR); Genard, Sylvie, 75012 Paris (FR)
(74) Mandataire: Dossmann, Gérard

(56) Documents cités:
- EP-A- 0 376 776
- DE-A- 2 524 329
- FR-A- 2 500 749

## Description

L'invention concerne l'utilisation de dérivés de quinoline- ou quinoxaline-5,8-diones ou -diols en cosmétique, notamment pour la coloration des matières kératiniques, et en particulier des cheveux. Elle concerne aussi des composés nouveaux de cette famille. L'invention vise également des compositions tinctoriales les contenant ainsi que le procédé de coloration de fibres kératiniques.

Dans la littérature, on connaît des produits d'addition de composés pyrroliques et de N,N-dialkylarylamines ou de N-alkylarylamines avec des quinoline-5,8-diones.

C'est ainsi que J. Griffiths et C. Blackburn, dans la revue *J. Chem*. *Res. (S)*, 1982, pages 320-321, ont étudié les réactions de la 1,4-naphtoquinone et de ses dérivés avec des N,N-dialkylarylamines.

Dans les articles de K. Yoshida et coll., *Bull. Chem. Soc. Jpn*, 1988, 61, pages 4335-4340, et *Chem. Lett.,* 1987, pages 1191-1194, il a été montré qu'en l'absence de sels métalliques, la réaction de la quinoline-5,8-dione avec des N,N-dialkylarylamines était lente et conduisait à des mélanges d'isomères 6- et 7-[p-(dialkylamino)phényl]quinoline-5,8-dione, mais qu'en revanche, en présence de sels métalliques, seuls les composés arylés en position 6 étaient obtenus. Les réactions sont généralement conduites dans l'acide acétique ; dans certains cas, un mélange chloroforme-éthanol/HCl peut également être utilisé d'après l'article de K. Yoshida et coll. dans *Chem*. *Lett*., 1991, 2027-2030.

K. Yoshida et coll. dans *J. Chem. Soc. Perkins Trans I*, 1992, 2713-2715 ont aussi étudié les dioxo-5,8-quinolylpyrroles pour leurs propriétés physiologiques et leur propriétés colorantes sur un support qui n'a pas été précisé. D'autre part, dans la revue *J. Chem*. *Soc*. *Perkins Trans I*, 1994, pages 2521-2523, K. Yoshida et coll se sont intéressés aux quinoline-5,8-diones portant un substituant en position 6 dans le but d'une utilisation comme précurseur de colorants utilisables en optoélectronique.

Par ailleurs, le seul dérivé connu de quinoxaline répond à la formule suivante :

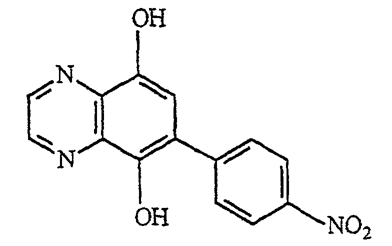

D'après ce qui précède, on connaît déjà des produits d'addition de composés pyrroliques et de N,N-dialkylarylamines ou de N-alkylarylamines avec des quinoline-5,8-diones mais aucune allusion n'est faite quant à leur application en cosmétique.

En revanche, les composés d'addition d'indoles avec les quinolinediones ou les quinoxalines sont nouveaux.

Dans le domaine de la teinture capillaire, on recherche des colorants qui doivent satisfaire à un certain nombre de critères, et notamment engendrer des colorations reproductibles avec des nuances riches et variées permettant d'obtenir une large palette de couleurs susceptible de satisfaire le formulateur.

La Demanderesse vient de découvrir qu'une nouvelle famille de dérivés de quinoline- ou quinoxaline-5,8-diones ou -diols pouvait être utilisée pour la coloration des matières kératiniques, et en particulier des cheveux. Cette nouvelle famille, objet de l'invention, satisfait à ces critères et est, de plus, très accessible car la plupart de ces colorants sont préparés en une seule étape dont la mise en oeuvre est aisée.

Les teintures obtenues à l'aide de ces colorants présentent des nuances reproductibles, puissantes et variées.

La présente invention a ainsi pour objet l'utilisation de composés de formules générales (I) et (II) définies ci-dessous, en teinture de matières kératiniques, et notamment des cheveux.

Un autre objet de la présente invention consiste en des composés nouveaux de cette famille.

L'invention a également pour objet des compositions tinctoriales et un procédé de teinture les mettant en oeuvre.

D'autres caractéristiques, aspects et avantages de l'invention apparaîtront encore plus clairement à la lecture de la description et des divers exemples qui suivent.

Un objet de la présente invention est l'utilisation en teinture de matières kératiniques, et notamment des cheveux, de composés de formules générales (I) et (II) : 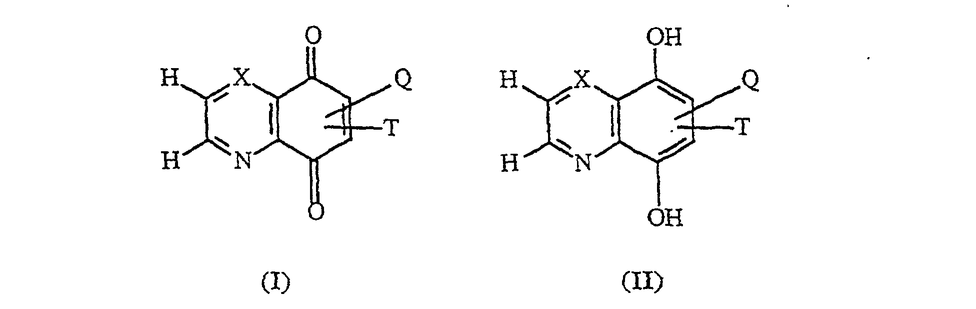 dans lesquelles :
Q représente les groupes 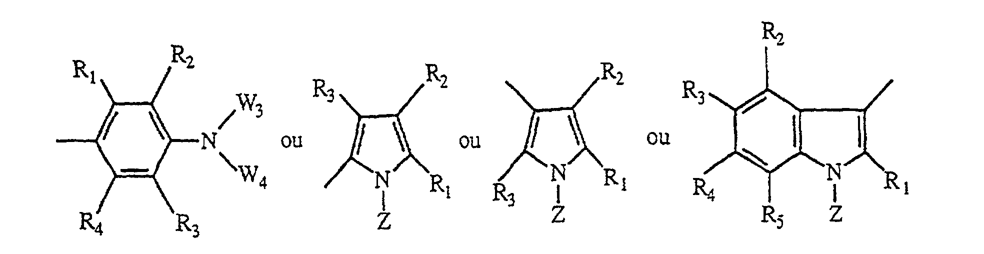
X représente le groupe CH ou un atome d'azote ;
T représente un atome d'hydrogène, un atome d'halogène ou un groupe hydroxyle ;
R₁, R₂, R₃, R₄ et R₅, identiques ou différents, sont choisis parmi un atome d'hydrogène, un groupe alcoxy en C₁-C₄, un groupe amino -N(W₁)(W₂), un groupe -NH-CO-W₁, un groupe -O-CO-W₁, un groupe hydroxyle, un groupe acide carboxylique ou ses dérivés tels qu'esters, amides ou sels minéraux ou d'amine organique, un atome d'halogène, un groupe alkyle en C₁-C₄, linéaire ou ramifié, éventuellement substitué par un groupe nitrile, par un groupe acide carboxylique ou ses dérivés tels qu'esters, amides ou sels minéraux ou d'amine organique, par un groupe hydroxyle, par un groupe tel que : 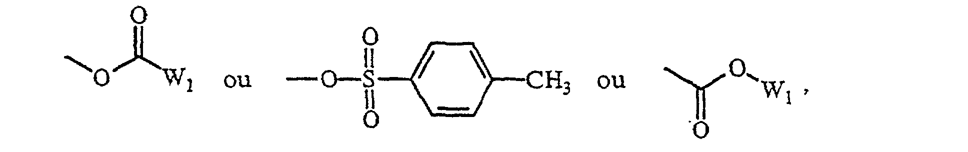 par un groupe éther ―OSi(W₁)₃, par un groupe amino 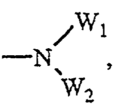 ou par un groupe amido 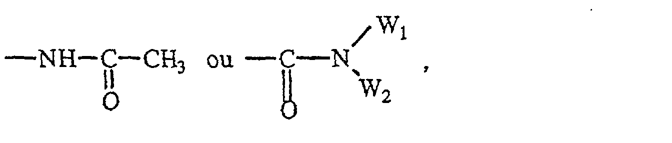
   Z représente un atome d'hydrogène, un groupe benzyle, un groupe phényle éventuellement substitué par un groupe hydroxyle, un groupe-O-CO-W₁, un groupe amino -N(W₁)(W₂), un groupe -NH-CO-W₁ ou un groupe nitrile ; un groupe alkyle en C₁-C₈, linéaire ou ramifié, éventuellement substitué par un groupe nitrile, par un groupe acide carboxylique ou ses dérivés tels qu'esters, amides ou sels minéraux ou d'amine organique, par un groupe hydroxyle, par un groupe tel que : 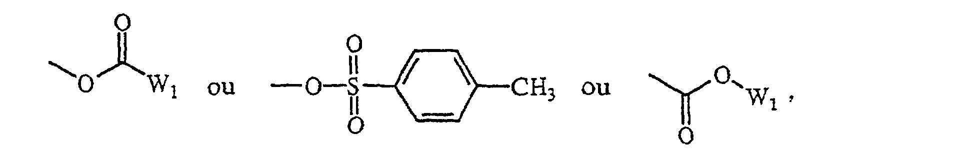 par un groupe éther ―OSi(W₁)₃, par un groupe amino 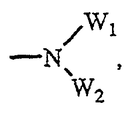 ou par un groupe amido 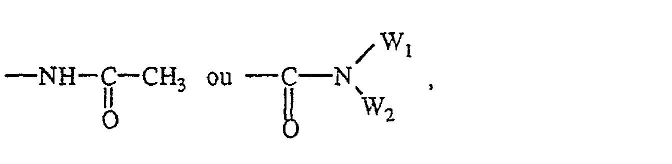
   W₁ et W₂, identiques ou différents, sont choisis parmi un atome d'hydrogène et un groupe alkyle en C₁-C₄ linéaire ou ramifié,
   W₃ et W₄, identiques ou différents, sont choisis parmi un atome d'hydrogène et un groupe alkyle en C₁-C₄, linéaire ou ramifié, éventuellement substitué par un ou plusieurs groupes hydroxyle, par un ou plusieurs groupes -CO-O-W₁ ou -O-CO-W₁, ou par un ou plusieurs groupes amino -N(W₁)(W₂).

Les groupes alkyle en C₁-C₄ ou les fragments alkyle des groupes alcoxy en C₁-C₄ peuvent être linéaires ou ramifiés, et choisis notamment parmi des groupes méthyle, éthyle, n-propyle, isopropyle, n-butyle, isobutyle, tert-butyle, de préférence parmi les groupes méthyle, éthyle et n-butyle.

Les groupes alkyle en C₁-C₈ peuvent être linéaires ou ramifiés ; ils peuvent être substitués par un groupe nitrile, un groupe acide carboxylique ou ses dérivés tels qu'esters, amides ou sels minéraux ou d'amine organique, un groupe hydroxyle, un groupe ester tel que alcoxycarbonyle, carboxy, alkylcarbonyloxy, formyloxy, tosyloxy, un groupe amino, un groupe amido tel que aminocarbonyle ou acétamido, un groupe éther choisi parmi les groupes trialkylsiloxy et siloxy.

Ces groupes alkyle en C₁-C₈ sont notamment choisis parmi des groupes méthyle, éthyle, n-propyle, isopropyle, n-butyle, isobutyle, tertbutyle, pentyle, hexyle, heptyle, octyle, de préférence parmi les groupes méthyle et pentyle.

Les atomes d'halogène sont choisis parmi le chlore, le brome, le fluor et l'iode, et en particulier il s'agit du chlore.

Parmi les composés de formules (I) et (II) définies ci-dessus, on peut notamment citer les composés suivants :
- 6-(1-pentyl-1H-pyrrol-2-yl)quinoline-5,8-dione,
- 6-(1-benzyl-1H-pyrrol-2-yl)quinoline-5,8-dione,
- 4-(5,8-dioxo-5,8-dihydro-quinolin-6-yl)-2,5-diméthyl-1H-pyrrole-3-carboxylate de méthyle,
- N-[2-[2-(5,8-dioxo-5,8-dihydro-quinolin-6-yl)pyrrol-1-yl]-éthyl] acétamide,
- 6-(1-phényl-1H-pyrrol-2-yl)quinoline-5,8-dione,
- 3-[2-(5,8-dioxo-5,8-dihydro-quinolin-6-yl)pyrrol-1-yl]-propionitrile,
- 6-chloro-7-(1-méthyl-1H-pyrrol-2-yl)quinoline-5,8-dione,
- 6-(1,2-diméthyl-1H-indol-3-yl)quinoline-5,8-dione,
- 6-(1-benzyl-1H-indol-3-yl)quinoline-5,8-dione,
- 6-(2-phényl-1H-indol-3-yl)quinoline-5,8-dione,
- 6-(1-méthyl-1H-indol-3-yl)quinoline-5,8-dione,
- 6-(2-méthyl-1H-indol-3-yl)quinoline-5,8-dione,
- 6-[p-(N,N-(2-diacétyloxyéthyl)amino)phényl]quinoline-5,8-dione,
- 6-(1-méthyl-1H-pyrrol-2-yl)quinoxaline-5,8-dione,
- 6-(1-méthyl-1H-pyrrol-2-yl)quinoline-5,8-dione,
- 7-(1-méthyl-1H-pyrrol-2-yl)quinoline-5,8-dione,
- 6-[p-(N,N-diéthylamino)phényl]quinoline-5,8-dione,
- 6-[p-(N,N-diméthylamino)phényl]quinoline-5,8-dione,
- 7-[p-(N,N-diméthylamino)phényl]quinoline-5,8-dione,
- 6-[4-(N,N-diéthylamino)-3-isopropénylphényl]quinoline-5,8-dione,
- 6-[4-(N,N-diéthylamino)-3-(N-acétylamino)phényl]quinoline-5,8-dione,
- 6-[4-(N,N-diméthylamino)-3-isopropénylphényl]quinoline-5,8-dione,
- 6-[4-(N,N-(di-n-butylamino)-3-hydroxyphényl]quinoline-5,8-dione,
- 6-[p-(N-n-butylamino)phényl]quinoline-5,8-dione,
- 6-[p-(N-méthylamino)phényl]quinoline-5,8-dione,
- 6-(1-méthyl-1H-pyrrol-2-yl)quinoline-5,8-diol,
- 7-(1-méthyl-1H-pyrrol-2-yl)quinoline-5,8-diol.

Parmi les composés de formules (I) et (II) utilisables dans la présente invention, on préfère plus particulièrement les dérivés de formule
(I) ou (II) pour laquelle X représente CH ; T représente H ; Q est choisi parmi : 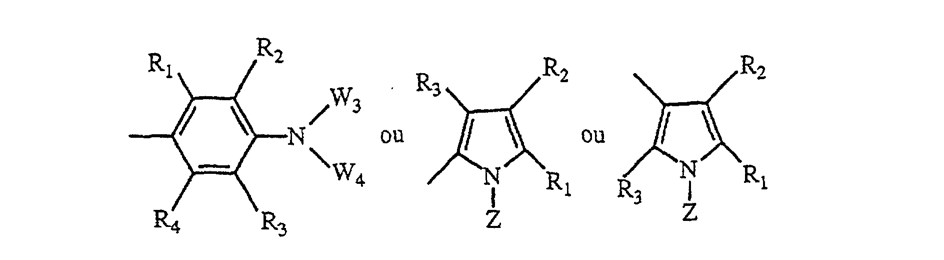 R₁ représente H, -OH ; R₂, R₃, R₄ et R₅ représentent chacun H ; Z représente H ou CH₃, phényle, benzyle ou 2-cyanoéthyle ; W₃ et W₄ sont choisis parmi H, les groupes méthyle, éthyle, n-butyle.

Les composés nouveaux répondent aux formules (I) et (II) de l'invention sous réserve que :
lorsque Q représente 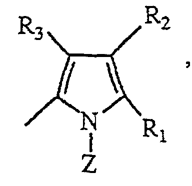 X = CH et T = H, alors Z est différent du groupe méthyle ;
lorsque Q représente 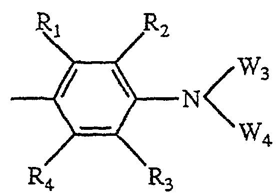 et l'un des substituants W₃ et W₄ représente un atome d'hydrogène, l'autre substituant ne représente pas un groupe méthyle ou n-butyle ; et
lorsque Q représente 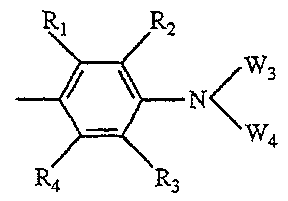 et les substituants W₃ et W₄ sont identiques, ils ne représentent pas un groupe méthyle, ni un groupe éthyle.

Ces composés peuvent être définis par les formules générales (III) et (IV) : 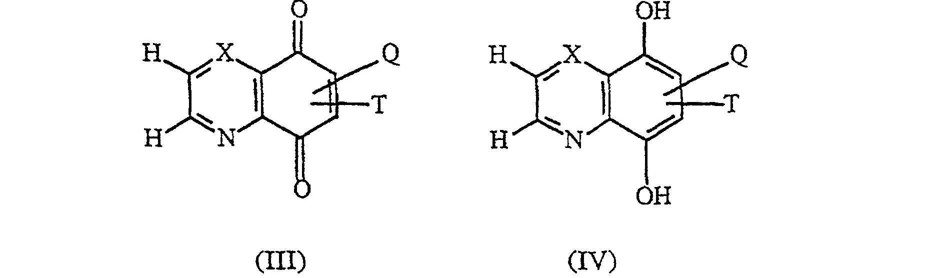 dans lesquelles Q représente : 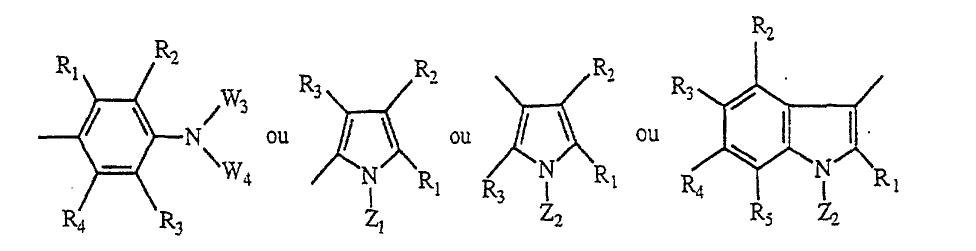
X représente le groupe CH ou un atome d'azote ;
T représente un atome d'hydrogène, un atome d'halogène ou un groupe hydroxyle ;
R₁, R₂, R₃, R₄ et R₅, identiques ou différents, sont choisis parmi un atome d'hydrogène, un groupe alcoxy en C₁-C₄, un groupe amino ―N(W₁)(W₂), un groupe -NH-CO-W₁, un groupe -O-CO-W₁, un groupe hydroxyle, un groupe acide carboxylique ou ses dérivés tels qu'esters, amides ou sels minéraux ou d'amine organique, un atome d'halogène, un groupe alkyle en C₁-C₄, linéaire ou ramifié, éventuellement substitué par un groupe nitrile, par un groupe acide carboxylique ou ses dérivés tels qu'esters, amides ou sels minéraux ou d'amine organique, par un groupe hydroxyle, par un groupe tel que : 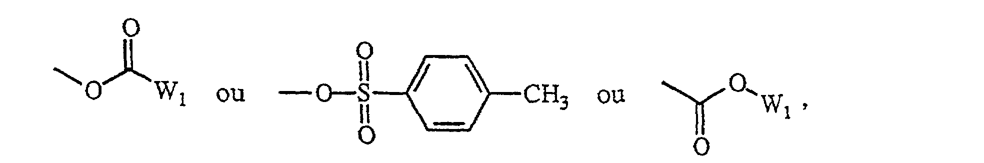 par un groupe éther ―OSi(W₁)₃, par un groupe amino 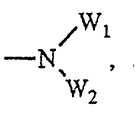 ou par un groupe amido 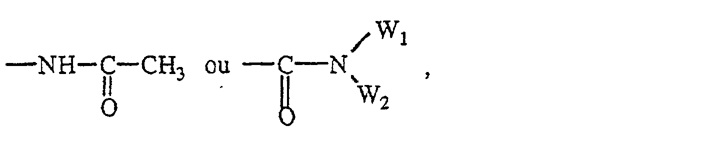
Z₂ représente un atome d'hydrogène, un groupe benzyle, un groupe phényle éventuellement substitué par un groupe hydroxyle, un groupe -CO-O-W₁ ou-O-CO-W₁, un groupe amino -N(W₁)(W₂), un groupe amido -CO-N(W₁)(W₂) ou -NH-CO-W₁ ou un groupe nitrile ; un groupe alkyle en C₁-C₈, linéaire ou ramifié, éventuellement substitué par un groupe nitrile, par un groupe acide carboxylique ou ses dérivés tels qu'esters, amides ou sels minéraux ou d'amine organique, par un groupe hydroxyle, par un groupe tel que : 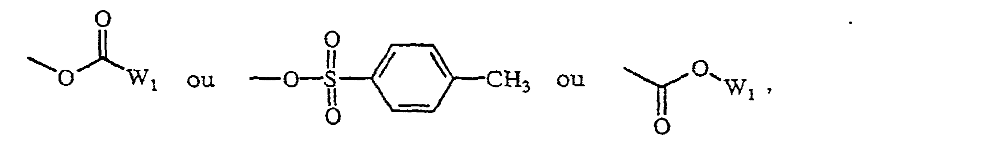 par un groupe éther ―OSi(W₁)₃, par un groupe amino 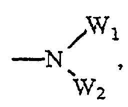 ou par un groupe amido 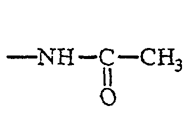 ou 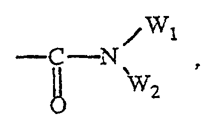
Z₁ a les mêmes significations que Z₂ sauf la signification méthyle dans les cas où T = H et X = CH,
W₁ et W₂, identiques ou différents, sont choisis parmi un atome d'hydrogène et un groupe alkyle en C₁-C₄ linéaire ou ramifié,
W₄ est choisi parmi un atome d'hydrogène et un groupe alkyle en C₁-C₄, linéaire ou ramifié, éventuellement substitué par un ou plusieurs groupes hydroxyle, par un ou plusieurs groupes -CO-O-W₁ ou -O-CO-W₁, ou par un ou plusieurs groupes amino -N(W₁)(W₂), et
W₃ a les mêmes significations que W₄ sous réserve que quand l'un des substituants W₃ et W₄ représente un atome d'hydrogène, l'autre substituant ne représente pas un groupe méthyle ou n-butyle ; et quand les substituants W₃ et W₄ sont identiques, ils ne représentent pas un groupe méthyle, ni un groupe éthyle.

Parmi les composés nouveaux de formules (III) et (IV) définis ci-dessus, on peut notamment citer les composés suivants :
- 6-(1-pentyl-1H-pyrrol-2-yl)quinoline-5,8-dione,
- 6-(1-benzyl-1H-pyrrol-2-yl)quinoline-5,8-dione,
- 4-(5,8-dioxo-5,8-dihydro-quinolin-6-yl)-2,5-diméthyl-1H-pyrrole-3-carboxylate de méthyle,
- N-[2-[2-(5,8-dioxo-5,8-dihydro-quinolin-6-yl)pyrrol-1-yl]-éthyl]acétamide,
- 6-(1-phényl-1H-pyrrol-2-yl)quinoline-5,8-dione,
- 3-[2-(5,8-dioxo-5,8-dihydro-quinolin-6-yl)pyrrol-1-yl]propionitrile,
- 6-chloro-7-(1-méthyl-1H-pyrrol-2-yl)quinoline-5,8-dione,
- 6-(1,2-diméthyl-1H-indol-3-yl)quinoline-5,8-dione,
- 6-(1-benzyl-1H-indol-3-yl)quinoline-5,8-dione,
- 6-[[p-(N,N-(2-diacétyloxyéthyl)amino]phényl]quinoline-5,8-dione,
- 6-(2-phényl-1H-indol-3-yl)quinoline-5,8-dione,
- 6-(1-méthyl-1H-indol-3-yl)quinoline-5,8-dione,
- 6-(2-méthyl-1H-indol-3-yl)quinoline-5,8-dione,
- 6-(1-méthyl-1H-pyrrol-2-yl)quinoxaline-5,8-dione.

Parmi les composés de formules (III) et (IV) utilisables dans la présente invention, on préfère plus particulièrement les dérivés de formule (III) ou (IV) pour laquelle X représente CH ou N; T représente H ; Q est choisi parmi : 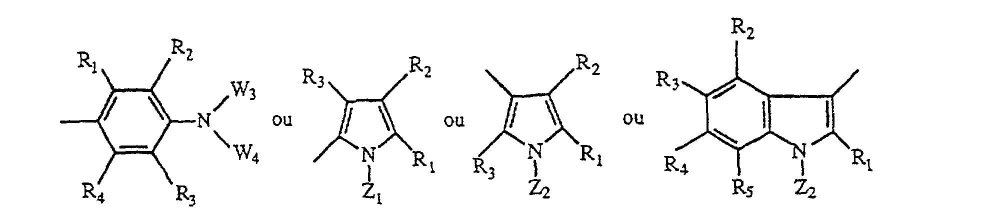 R₁ est choisi parmi H et le groupe -CH₃ ; R₂ représente H, le groupe ―COO―W₁, R₃ représente H, le groupe méthyle, R₄ et R₅ représentent chacun H ; W₁ représente le groupe -CH₃ ; Z₁ représente -C₅H₁₁, -(CH₂)₂-NH-COCH₃, -(CH₂)₂-CN, phényle ; Z₂ représente H, les groupes -CH₃ et benzyle ; W₃ et W₄ peuvent représenter le groupe ―(CH₂)₂OCOW₁.

Les inventeurs ont découvert qu'il était possible de teindre les fibres kératiniques, et en particulier les cheveux, grâce aux composés de formules I et II. Les couleurs obtenues sont fonction de la nature des substituants Q et T utilisés pour réaliser la synthèse. En faisant varier la nature de ces substituants, il est possible de réaliser une gamme de couleurs variées.

Conformément à l'invention, les composés de formule (I) ou (II) sont utilisés pour la teinture directe encore appelée coloration semi-permanente de fibres kératiniques, et en particulier de cheveux.

Les composés de formule (I) ou (II) peuvent être introduits, à titre de colorants directs, dans des compositions de teinture d'oxydation pour enrichir de reflets les teintures obtenues au moyen des précurseurs de colorants d'oxydation et éventuellement des coupleurs généralement utilisés dans ce type de teinture.

Les composés de formule (II) sont susceptibles d'être oxydés par l'oxygène de l'air et sont utilisés pour la teinture des fibres kératiniques, en particulier des cheveux, selon le procédé dit de teinture progressive consistant à appliquer le composé de formule (II) sur les fibres, à le laisser poser à l'air de 5 à environ 45 minutes, et de préférence de 5 à environ 30 minutes, sur lesdites fibres et à répéter cette opération le nombre de fois souhaité jusqu'à avoir de façon préférée la coloration souhaitée.

L'invention a également pour objet une composition tinctoriale, en particulier cosmétique, pour matières kératiniques, en particulier pour des fibres kératiniques humaines, comprenant dans un milieu approprié pour la teinture, une quantité efficace d'au moins un des composés de formule (I) ou (II) définie ci-dessus.

Au sens de l'invention, on entend principalement par matières kératiniques, les fibres textiles naturelles telles que la laine, la fourrure animale, par matières kératiniques humaines, la peau et les ongles, et par fibres kératiniques humaines, les cheveux, les poils, les cils et les sourcils. L'invention porte plus particulièrement encore sur les cheveux.

Les composés de formule (I) ou (II) sont généralement présents dans des proportions comprises entre environ 0,01 et 10 %, de préférence entre environ 0,05 et 5 % en poids, par rapport au poids total de la composition de teinture.

Le milieu cosmétiquement acceptable est de préférence un milieu constitué par de l'eau et/ou des solvants organiques acceptables sur le plan cosmétique si la composition est destinée à être utilisée en cosmétique, et plus particulièrement, des alcools (l'alcool éthylique, l'alcool isopropylique, l'alcool benzylique), des glycols ou éthers de glycols (le propylèneglycol ou ses éthers tels que, par exemple, le monométhyléther de propylèneglycol, le butylèneglycol, le dipropylèneglycol ainsi que les alkyléthers de diéthylèneglycol comme par exemple le monoéthyléther ou le monobutyléther de diéthylèneglycol, les éthers monométhylique, monoéthylique et monobutylique d'éthylèneglycol) dans des concentrations comprises entre 0,5 et 25 %, de préférence, entre environ 2 et 15 % en poids par rapport au poids total de la composition. Le milieu cosmétiquement acceptable peut aussi contenir des corps gras tels que des huiles et des cires.

On peut également ajouter à la composition selon l'invention des amides gras tels que les mono- et di-éthanolamides et des acides dérivés du coprah, de l'acide laurique ou de l'acide oléique, à des concentrations comprises entre environ 0,05 et 10 % en poids.

La composition tinctoriale selon l'invention peut encore contenir, pour obtenir des teintes variées, outre les colorants de formule (I) ou (II), d'autre(s) colorant(s) direct(s) classiquement utilisés, et en particulier des colorants nitrés benzéniques, comme les nitrophénylènediamines, les nitrodiphénylamines, les nitroanilines, les éthers de phénol nitrés ou les nitrophénols, des nitropyridines, des colorants anthraquinoniques, mono- ou di-azoïques, triarylméthaniques, aziniques, acridiniques et xanthéniques, ou encore des colorants métallifères. La proportion de tous ces autres colorants directs d'addition peut varier entre 0,05 et 10 % en poids par rapport au poids total de la composition tinctoriale.

Ladite composition tinctoriale peut contenir en outre tout autre adjuvant utilisé habituellement en teinture des matières kératiniques, et par exemple, des agents tensioactifs bien connus de l'art antérieur et de type anionique, cationique, non-ionique, amphotère, zwittérionique ou leurs mélanges, des agents épaississants, des agents anti-oxydants, des parfums, des agents séquestrants, des agents dispersants, des agents de conditionnement, des agents conservateurs, des agents opacifiants, etc...

Bien entendu, l'homme de l'art veillera à choisir le ou les éventuels composés complémentaires mentionnés ci-dessus, de telle manière que les propriétés avantageuses liées intrinsèquement à la composition tinctoriale selon l'invention ne soient pas, ou substantiellement pas, altérées par la ou les adjonctions envisagées.

La composition de teinture selon l'invention peut être formulée à pH acide, neutre ou alcalin, le pH pouvant varier par exemple de 2 à 11, et de préférence de 2,5 à 10, et pouvant être ajusté au moyen d'agents d'alcalinisation ou d'agents d'acidification antérieurement bien connus.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 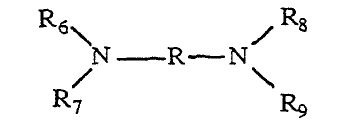 dans laquelle R représente un groupe propylène éventuellement substitué par un groupe hydroxyle ou un groupe alkyle en C₁-C₄ ; R₆, R₇, R₈ et R₉, identiques ou différents, représentent un atome d'hydrogène, un groupe alkyle en C₁-C₄ ou hydroxyalkyle en C₁-C₄.

Les agents acidifiants utilisés sont classiquement connus. On peut citer, à titre d'exemple, des acides minéraux ou organiques comme l'acide chlorhydrique, l'acide orthophosphorique, des acides carboxyliques comme l'acide tartrique, l'acide citrique, l'acide lactique ou des acides sulfoniques.

Lorsque la composition est destinée à être appliquée sur les fibres kératiniques humaines, elle peut se présenter sous des formes diverses, telles que sous forme de liquide, de crème, de gel ou toute autre forme appropriée pour réaliser une coloration des fibres kératiniques. En particulier, elle peut être conditionnée sous pression en flacon aérosol en présence d'un agent propulseur et former une mousse.

Un autre objet de la présente invention porte sur un procédé de teinture des fibres kératiniques humaines, et plus particulièrement des cheveux, par coloration directe, consistant à laisser agir une composition tinctoriale renfermant au moins un composé de formule (I) ou (II), sur les fibres kératiniques sèches ou humides. On peut utiliser la composition selon l'invention en tant que composition non rincée, c'est-à-dire qu'après application de la composition sur les fibres, on sèche sans rinçage intermédiaire. Dans les autres modes d'application, on laisse agir la composition sur les fibres pendant un temps de pose variant entre 3 et 60 minutes environ, de préférence entre 5 et 45 minutes, on rince, éventuellement on lave et on rince à nouveau, puis on sèche.

Un objet supplémentaire de la présente invention concerne un procédé de teinture progressive, qui consiste à appliquer une composition tinctoriale renfermant un composé de formule (II) définie ci-dessus sur des fibres kératiniques, à laisser poser ladite composition à l'air pendant 5 à environ 45 minutes, et de préférence de 5 à environ 30 minutes, puis à rincer les fibres, éventuellement les laver, les rincer alors à nouveau, puis à les sécher.

On va maintenant donner, à titre d'illustration, et sans aucun caractère limitatif, plusieurs exemples de préparation de composés de formule (I) ou (II) selon l'invention, ainsi que des exemples concrets de compositions tinctoriales à base de tels composés.

Pour préparer les composés de formules (I) à (IV), on utilise généralement le protocole opératoire décrit par K. Yoshida, Y. Ueno, M. Suzuki, Y. Yoshida, et Y. Kubo, dans *J*. *Chem. Soc*. *Perkins Trans I*, 1992, 2713-2715, par réaction de quinoline-5,8-dione sur un dérivé pyrrolique et en choisissant un rapport molaire convenable entre les deux réactifs en milieu solvant.

### EXEMPLES DE PRÉPARATION

On a préparé les composés des exemples suivants à l'aide du protocole opératoire général décrit par K. Yoshida, Y. Ueno, M. Suzuki, Y. Yoshida, et Y. Kubo, dans *J. Chem*. *Soc*. *Perkins Trans I,* 1992, 2713-2715. On a utilisé ce protocole opératoire pour les exemples 1 à 7.

A une solution de 3,14 mmoles de chlorure ferrique dans 60 ml de solution aqueuse d'acide acétique à 20 % et à température ambiante, on ajoute un dérivé pyrrolique (25,12 mmoles) et la quinoline-5,8-dione dans 50 ml de chloroforme. Le milieu réactionnel est maintenu sous vive agitation et l'évolution de la réaction est suivie par chromatographie sur couche mince. En fin de réaction, la phase organique est séparée et la phase aqueuse est extraite par 2 x 30 ml de chloroforme. Les phases organiques réunies sont lavées avec une solution aqueuse de carbonate de sodium puis avec de l'eau avant d'être séchées sur du sulfate de magnésium, filtrées et concentrées sous pression réduite. Le produit brut ainsi obtenu est purifié par chromatographie sur colonne de gel de silice.

### Exemple 1 : préparation de la 6-(1-méthyl-1H-pyrrol-2-yl)quinoline-5,8-dione.

On prépare le composé de l'exemple 1 en suivant le protocole opératoire décrit ci-dessus. Au bout de 5 minutes de réaction, on isole la 6-(1-méthyl-1H-pyrrol-2-yl)quinoline-5,8-dione. Le rendement est de 62 %.

On a vérifié la structure du composé par spectroscopie RMN du ¹H et du ¹³C.
RMN ¹H, 200 MHz (CDCl₃, δ ppm) : 3,73 (s, 3H), 6,30 (d.d, 1H), 6,77 (d.d, 1H), 6,94 (m,1H), 7,03 (s, 1H), 7,70 (d.d, 1H), 8,49 (d, 1H), 9,05 (d, 1H). RMN ¹³C, 50 MHz (CDCl₃, δ ppm) : 36,4 ; 109,9 ; 118,5 ; 125,9 ; 127,4 ; 129,5 ; 130,2 ; 131,0 ; 135,1; 136,4 ; 138,6 ; 154,6 ; 183,0 ; 183,9.
Point de fusion : 167-168 °C.

### Exemple 2 : préparation de la 6-(1-pentyl-1H-pyrrol-2-yl)quinoline-5,8-dione.

Au bout de 5 minutes de réaction, on isole selon le protocole précédent la 6-(1-pentyl-1H-pyrrol-2-yl)quinoline-5,8-dione sous la forme d'une huile. Le rendement est de 18%.
RMN ¹H, 200 MHz (CDCl₃, δ ppm) : 0,83 (t, 3H), 1,22 (m, 4H), 1,73 (q, 2H), 3,96 (t, 2H), 6,30 (d.d, 1H), 6,67 (d.d, 1H), 7,00 (m, 2H), 7,70 (d.d, 1H), 8,49 (d.d, 1H), 9,05 (d.d, 1H).

### Exemple 3 : préparation de la 6-(1-benzyl-1H-pyrrol-2-yl)quinoline-5,8-dione.

Au bout de 5 minutes de réaction, on isole selon le protocole précédent la 6-(1-benzyl-1H-pyrrol-2-yl)quinoline-5,8-dione. Le rendement est de 53 %.
RMN¹H, 200MHz (CDCl₃, δ ppm) : 5,21 (s, 2H), 6,36 (d.d, 1H), 6,73 (d.d, 1H), 6,92 (s,1H), 6,93-7,01 (m, 3H), 7,18-7,24 (m, 3H), 7,68 (d.d, 1H), 8,47 (d.d, 1H), 9,30 (d.d, 1H).
RMN¹³C, 50 MHz (CDCl₃, δ ppm): 52,3 ; 110,0 ; 118,0 ; 126,0 ; 126,7 ; 127,4 ; 127,9 ; 128,8 ; 129,3 ; 129,5 ; 131,9 ; 135,0 ; 137,1; 139,4; 147,4 ; 154,5 ; 183,0 ; 183,9.
Point de fusion : 172-173 °C.

### Exemple 4 : préparation du 4-(5,8-dioxo-5,8-dihydro-quinolin-6-yl)-2,5-diméthyl-1H-pyrrole-3-carboxylate de méthyle.

Au bout de 5 heures de réaction, on isole selon le protocole précédent le 4-(5,8-dioxo-5,8-dihydro-quinolin-6-yl)-2,5-diméthyl-1H-pyrrole-3-carboxylate de méthyle. Le rendement est de 58 %.
RMN¹H, 200 MHz (CDCl₃, δ ppm) : 2,25 (s, 3H), 2,52 (s, 3H), 3,63 (s, 3H), 6,94 (s, 1H), 7,70 (d.d, 1H), 8,36 (m, 1H), 8,48 (d.d, 1H), 9,05 (d.d, 1H).
Point de fusion : 237-241 °C.

### Exemple 5 : préparation du N-[2-[2-(5,8-dioxo-5,8-dihydro-quinolin-6-yl)-pyrrol-1-yl]-éthyl]acétamide.

Au bout de 20 minutes de réaction, on isole selon le protocole précédent le N-[2-[2-(5,8-dioxo-5,8-dihydro-quinolin-6-yl)-pyrrol-1-yl]-éthyl]acétamide. Le rendement est de 41 %.
RMN¹H, 200 MHz (CDCl₃, δ ppm) : 1,89 (s, 3H), 3,51 (m, 2H), 4,13 (t, 2H), 5,90 (s large, 1H), 6,33 (d.d, 1H), 6,61 (d.d, 1H), 7,00-7,03 (m,2H), 7,71 (d.d, 1H), 8,48 (d.d, 1H), 9,04 (d.d, 1H).
Point de fusion: 164-165 °C.

### Exemple 6 : préparation de la 6-(1-phényl-1H-pyrrol-2-yl)quinoline-5,8-dione.

Au bout de 6 heures de réaction, on isole selon le protocole précédent la 6-(1-phényl-1H-pyrrol-2-yl)quinoline-5,8-dione. Le rendement est de 64%.
RMN¹H, 200 MHz (CDCl₃, δ ppm) : 6,44-6,48 (m, 2H), 6,93 (d.d, 1H), 7,31-7,47 (m, 6H), 7,81 (d.d, 1H), 8,27 (d.d, 1H), 8,99 (d.d, 1H).
RMN¹³C, 50MHz (CDCl₃, δ ppm) : 111,1 ; 120,4 ; 125,0 ; 125,1 ; 127,4 ; 127,8 129,5 ; 129,8 ; 130,2 ; 131,7 ; 135,0 ; 138,3 ; 139,9 ; 147,4 ; 154,5 ; 182,7 ; 183,8.
Point de fusion : 132-134 °C.

### Exemple 7 : préparation du 3-[2-(5,8-dioxo-5,8-dihydro-quinolin-6-yl)-pyrrol-1-yl]propionitrile.

Au bout de 30 minutes de réaction, on isole selon le protocole précédent le 3-[2-(5,8-dioxo-5,8-dihydro-quinolin-6-yl)-pyrrol-1-yl]-propionitrile. Le rendement est de 44 %.
RMN¹H, 200MHz (CDCl₃, δ ppm) : 2,88 (t, 2H), 4,22 (t, 2H), 6,37 (d.d, 1H), 6,59 (d.d, 1H), 7,08-7,11 (m,2H), 7,73 (d.d, 1H), 8,50 (d.d, 1H), 9,08 (d.d, 1H).
RMN ¹³C, 50MHz (CDCl₃, δ ppm) : 20,2 ; 44,0 ; 111,2 117,0 ; 117,4 ; 126,1 ; 127,6; 127,7 ; 129,2 ; 134,2 ; 135,2 ; 139,4; 147,5 ; 154,9 ; 182,9 ; 184,1.
Point de fusion : 166-167 °C.

### Exemple 8 : préparation du 6-(1-méthyl-1H-pyrrol-2-yl)quinoline-5,8-diol.

On a préparé le 6-(1-méthyl-1H-pyrrol-2-yl)quinoline-5,8-diol selon le protocole décrit par K. Yoshida et coll. dans l'article *J. Chem*. *Perkin Trans I,* 1994, 2521-2523.
Point de fusion : 142-143 °C.

### Exemple 9 : Préparation de la 6-chloro-7-(1-méthyl-1H-pyrrol-2-yl)quinoline-5,8-dione.

On agite à température ambiante pendant 24 heures une solution de 2,58 mmoles de 6-chloroquinoline-5,8-dione avec 20,7 mmoles de 1-méthylpyrrole dans 50 ml d'acide acétique. Le milieu est ensuite concentré sous pression réduite, extrait par du chloroforme et les phases organiques réunies sont lavées successivement avec une solution aqueuse de carbonate de sodium, puis de l'eau avant d'être séchées sur du sulfate de magnésium, filtrées et concentrées sous vide. Le produit brut obtenu est purifié par chromatographie sur colonne de gel de silice pour conduire à la 6-chloro-7-(1-méthyl-1H-pyrrol-2-yl)quinoline-5,8-dione, avec un rendement de 40 %.
RMN¹H, 200 MHz (CDCl₃, δ ppm) : 3,57 (s, 3H), 6,34 (d.d, 1H), 6,61 (d.d, 1H), 6,94 (t, 1H), 7,75 (d.d, 1H), 8,56 (d.d, 1H), 9,10 (d.d, 1H).
Point de fusion : 168-169 °C.

Les exemples 10 à 14 décrivent la condensation des indoles sur le noyau quinoline-5,8-dione. La monocondensation a été vérifiée par spectrométrie de masse.

### Exemple 10 : Préparation de la 6-(1,2-diméthyl-1H-indol-3-yl)quinoline-5,8-dione.

Le produit est préparé selon le protocole général des exemples 1 à 7 avec un rendement de 46 %.
Point de fusion : 213-214 °C.

### Exemple 11 : Préparation de la 6-(1-benzyl-1H-indol-3-yl)quinoline-5,8-dione.

Le produit est préparé selon le protocole général avec un rendement de 51 %.
Point de fusion : 179-180 °C.

### Exemple 12 : Préparation de la 6-(2-phényl-1H-indol-3-yl)quinoline-5,8-dione.

Le produit est préparé selon le protocole général avec un rendement de 62 %.
Point de fusion : 330 °C (décomposition).

### Exemple 13 : Préparation de la 6-(1-méthyl-1H-indol-3-yl)quinoline-5,8-dione.

Le produit est préparé selon le protocole général avec un rendement de 54 %.
Point de fusion : 215-217 °C.

### Exemple 14 : Préparation de la 6-(2-méthyl-1H-indol-3-yl)quinoline-5,8-dione.

Le produit est préparé selon le protocole général avec un rendement de 58 %.
Point de fusion : 221-223 °C.

Le mode opératoire utilisé pour la préparation des 6-[p-(dialkylamino)phényl]quinoline 5,8-diones des exemples 15 et 16 est celui décrit par K. YOSHIDA et coll. dans l'article *Bull*. *Chem. Soc. Japn*., 1988, 61, 4335.

### Exemple 15 : Préparation de la 6-(p-(diéthylamino)phényl)quinoline-5,8-dione.

Le produit est préparé selon le protocole ci-dessus avec un rendement de 78 %.
RMN¹H, 200 MHz (DMSO d₆, δ ppm) : 1,20 (t, 6H), 3,44 (q, 4H), 6,72 (d, 2H), 7,18 (s, 1H), 7,62 (d, 2H), 7,62 (d.d, 1H), 8,50 (d.d, 1H), 9,04 (d.d, 1H).
Point de fusion : 136-137 °C.

### Exemple 16 : préparation de la 6-[(p-(N,N-(2-diacétyloxyéthyl)-amino)phényl]quinoline-5,8-dione.

Le produit est préparé selon le protocole ci-dessus avec un rendement de 64 %. Il est isolé sous la forme d'une huile visqueuse violet sombre.
RMN¹H, 200MHz (DMSO d₆, δ ppm) : 2,53 (s, 6H), 3,62-3,68 (m, 4H), 4,24-4,27 (m, 4H), 6,76-6,88 (m, 2H), 7,11-7,26 (m, 2H), 7,59-7,72 (m, 2H), 8,49-8,53 (m, 1H), 9,06 (m, 1H).

### Exemple 17 : préparation de la 6-(1-méthyl-1H-pyrrol-2-yl)quinoxaline-5,8-dione.

A une solution de 338 mg de FeCl₃, 6H₂O dans un mélange eau (48 ml)/acide acétique (12 ml), on ajoute rapidement 0,81 g de N-méthyl-pyrrole et 200 mg de quinoxaline-5,8-dione en solution dans 50 ml de chloroforme. Le milieu est agité vivement pendant 10 minutes puis la phase organique est séparée.

On extrait la phase aqueuse deux fois avec du chloroforme et les phases organiques réunies sont lavées avec une solution aqueuse de carbonate de sodium, puis de l'eau, avant d'être séchées sur du sulfate de magnésium, filtrées et concentrées sous vide. Le résidu ainsi obtenu est purifié par chromatographie sur colonne de gel de silice pour conduire à la 6-(1-méthyl-1H-pyrrol-2-yl)quinoxaline-5,8-dione avec un rendement de 68 %.
RMN¹H, 200MHz (CDCl₃, δ ppm): 3,77 (s, 3H), 6,34 (d.d, 1H), 6,88 (d.d, 1H), 6,99 (m, 1H), 7,13 (s, 1H), 9,05 (s, 2H).
Point de fusion : 161-162 °C.

### EXEMPLES DE COMPOSITIONS TINCTORIALES

### Exemples de coloration à pH acide

La formulation générale à pH acide est la suivante :

| | Quantité |
|---|---|
| Composé d'exemple de préparation (voir tableau 1) | 2,24x10⁻³ mole |
| Alcool benzylique | 10 g |
| Alcool éthylique | 21 g |
| Glycérol | 5 g |
| Hydroxyéthylcellulose vendu par la société Union Carbide sous le nom de CELLOSIZE QP 4400 H. | 2,3 g |
| Acide citrique | 1,4 g |
| eau qsp | 100 g |

Le protocole de teinture consiste à appliquer à température ambiante la préparation dont le pH est aux environs de 3, sur des cheveux gris naturels permanentés ou non, ou sur des cheveux décolorés, à raison de 3 grammes par gramme de cheveux. Après avoir laissé poser la composition pendant 30 minutes, on a rincé puis séché les mèches. Les résultats de coloration sont regroupés dans le tableau 1.

**Tableau 1**

| Composé étudié | Masse en g% | Cheveux gris naturels | Cheveux décolorés | Cheveux gris permanentés |
|---|---|---|---|---|
| Composé de l'ex. 3 | 0,704 | | bois de rose | |
| Composé de l'ex. 15 | 0,686 | bleu | bleu | bleu |
| Composé de l'ex. 4 | 0,695 | | bois de rose | |
| Composé de l'ex. 5 | 0,693 | | framboise | |
| Composé de l'ex. 6 | 0,673 | bois de rose | rouge orangé | bois de rose |
| Composé de l'ex. 7 | 0,621 | | rose léger | |
| Composé de l'ex. 9 | 0,611 | | beige rosé | |
| Composé de l'ex. 1 | 0,534 | bois de rose | rouge violacé | bois de rose |

### Exemples de coloration à pH 7,5

La formulation générale à pH 7,5 est la suivante :

| | Quantité |
|---|---|
| Composé d'exemple de préparation (voir tableau 2) | 2,24x10⁻³ mole |
| Alcool benzylique | 10 g |
| Alcool éthylique | 21 g |
| Glycérol Hydroxyéthylcellulose vendu par la société Union Carbide sous le nom de | 5 g |
| CELLOSIZE QP 4400 H. | 2,3 g |
| Tampon K₂HPO₄/KH₂HPO₄ (1,5 M/1 M) | 10 g |
| eau qsp | 100 g |

Le protocole de teinture consiste à appliquer à température ambiante la préparation, dont le pH est aux environs de 7,5, sur des cheveux gris naturels permanentés ou non, ou sur des cheveux décolorés, à raison de 3 grammes par gramme de cheveux. Après avoir laissé poser la composition pendant 30 minutes, on a rincé puis séché les mèches. Les résultats de coloration sont regroupés dans le tableau 2.

**Tableau 2**

| Composé étudié | Masse en g% | Cheveux gris naturels | Cheveux décolorés | Cheveux gris permanentés |
|---|---|---|---|---|
| Composé de l'ex. 15 | 0,686 | bleu vert | bleu | bleu |
| Composé de l'ex. 1 | 0,534 | bois de rose | rouge violacé | bois de rose |

### Exemples de coloration à pH basique

La formulation générale à pH 8,6 est la suivante :

| | Quantité |
|---|---|
| Composé d'exemple de préparation (voir tableau 3) | 2,24x10⁻³ mole |
| Alcool benzylique | 10 g |
| Alcool éthylique | 21 g |
| Glycérol Hydroxyéthylcellulose vendu par la société Union Carbide sous le nom de | 5 g |
| CELLOSIZE QP 4400 H. | 2,3 g |
| Tampon aminopropanediol/HCl (1 M/0,035 M) | 10 g |
| eau qsp | 100 g |

Le protocole de teinture consiste à appliquer à température ambiante la préparation, dont le pH est aux environs de 8,6, sur des cheveux gris naturels permanentés ou non, ou sur des cheveux décolorés, à raison de 3 grammes par gramme de cheveux. Après avoir laissé poser la composition pendant 30 minutes, on a rincé puis séché les mèches. Les résultats de coloration sont regroupés dans le tableau 3.

**Tableau 3**

| Composé étudié | Masse en g% | Cheveux gris naturels | Cheveux décolorés | Cheveux gris permanentés |
|---|---|---|---|---|
| Composé de l'ex. 15 | 0,686 | bleu vert | bleu | bleu |
| Composé de l'ex. 1 | 0,534 | bois de rose | rouge violacé | bois de rose |

## Revendications

1. Utilisation pour la coloration de matières kératiniques, en particulier de fibres kératiniques humaines, de composés de formules générales (I) et (II) : 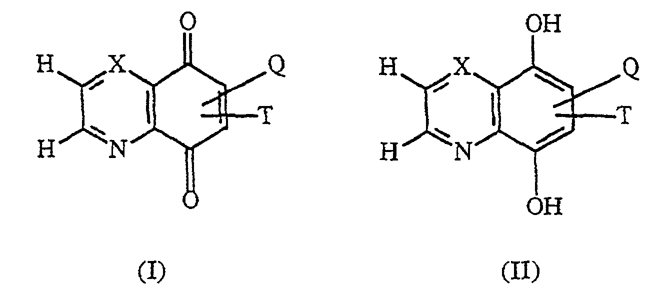 dans lesquelles :
Q représente les groupes 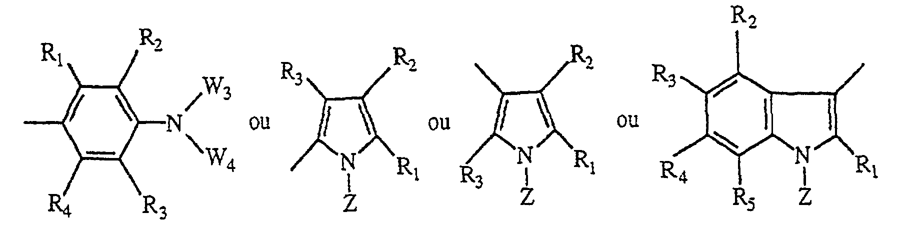
X représente le groupe CH ou un atome d'azote ;
T représente un atome d'hydrogène, un atome d'halogène ou un groupe hydroxyle ;
R₁, R₂, R₃, R₄ et R₅, identiques ou différents, sont choisis parmi un atome d'hydrogène, un groupe alcoxy en C₁-C₄, un groupe amino -N(W₁)(W₂), un groupe -NH-CO-W₁, un groupe -O-CO-W₁, un groupe hydroxyle, un groupe acide carboxylique ou ses dérivés tels qu'esters, amides ou sels minéraux ou d'amine organique, un atome d'halogène, un groupe alkyle en C₁-C₄, linéaire ou ramifié, éventuellement substitué par un groupe nitrile, par un groupe acide carboxylique ou ses dérivés tels qu'esters, amides ou sels minéraux ou d'amine organique, par un groupe hydroxyle, par un groupe tel que : 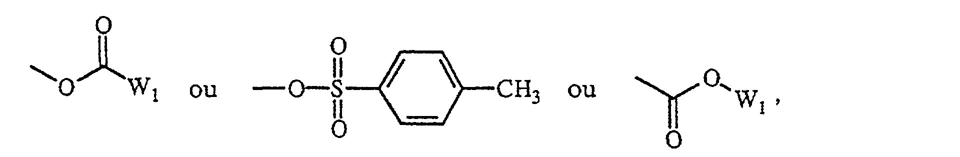 par un groupe éther ―OSi(W₁)₃, par un groupe amino 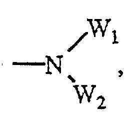 ou par un groupe amido 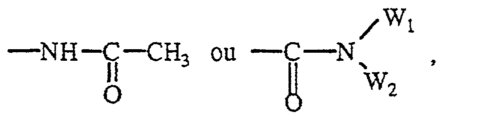
Z représente un atome d'hydrogène, un groupe benzyle, un groupe phényle éventuellement substitué par un groupe hydroxyle, un groupe -O-CO-W₁, un groupe amino -N(W₁)(W₂), un groupe-NH-CO-W₁ ou un groupe nitrile ; un groupe alkyle en C₁-C₈, linéaire ou ramifié, éventuellement substitué par un groupe nitrile, par un groupe acide carboxylique ou ses dérivés tel qu'esters, amides ou sels minéraux ou d'amine organique, par un groupe hydroxyle, par un groupe tel que : 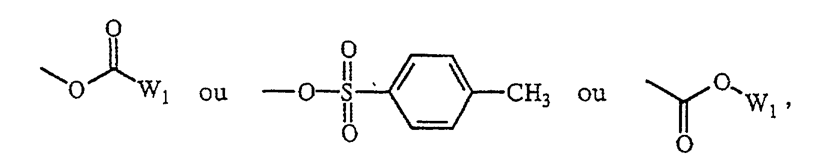 par un groupe éther ―OSi(W₁)₃, par un groupe amino 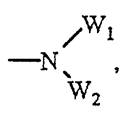 ou par un groupe amido 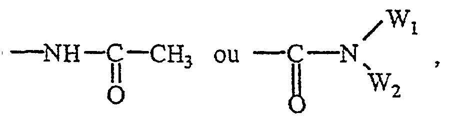
W₁ et W₂, identiques ou différents, sont choisis parmi un atome d'hydrogène et un groupe alkyle en C₁-C₄ linéaire ou ramifié,
W₃ et W₄, identiques ou différents, sont choisis parmi un atome d'hydrogène et un groupe alkyle en C₁-C₄, linéaire ou ramifié, éventuellement substitué par un ou plusieurs groupes hydroxyle, par un ou plusieurs groupes -CO-O-W₁ ou-O-CO-W₁, ou par un ou plusieurs groupes amino-N(W₁)(W₂).

2. Utilisation selon la revendication 1, caractérisée par le fait qu'il s'agit de cheveux.

3. Utilisation selon l'une quelconque des précédentes revendications, caractérisée par le fait que les composés de formule (I) ou (II) sont choisis parmi les composés suivants :
- 6-(1-pentyl-1H-pyrrol-2-yl)quinoline-5,8-dione,
- 6-(1-benzyl-1H-pyrrol-2-yl)quinoline-5,8-dione,
- 4-(5,8-dioxo-5,8-dihydro-quinolin-6-yl)-2,5-diméthyl-1H-pyrrole-3-carboxylate de méthyle,
- N-[2-[2-(5,8-dioxo-5,8-dihydro-quinolin-6-yl)pyrrol-1-yl]-éthyl]acétamide,
- 6-(1-phényl-1H-pyrrol-2-yl)quinoline-5,8-dione,
- 3-[2-(5,8-dioxo-5,8-dihydro-quinolin-6-yl)pyrrol-1-yl]propionitrile,
- 6-chloro-7-(1-méthyl-1H-pyrrol-2-yl)quinoline-5,8-dione,
- 6-(1,2-diméthyl-1H-indol-3-yl)quinoline-5,8-dione,
- 6-(1-benzyl-1H-indol-3-yl)quinoline-5,8-dione,
- 6-(2-phényl-1H-indol-3-yl)quinoline-5,8-dione,
- 6-(1-méthyl-1H-indol-3-yl)quinoline-5,8-dione,
- 6-(2-méthyl-1H-indol-3-yl)quinoline-5,8-dione,
- 6-[p-(N,N-(2-diacétyloxyéthyl)amino)phényl]quinoline-5,8-dione,
- 6-(1-méthyl-1H-pyrrol-2-yl)quinoxaline-5,8-dione,
- 6-(1-méthyl-1H-pyrrol-2-yl)quinoline-5,8-dione,
- 7-(1-méthyl-1H-pyrrol-2-yl)quinoline-5,8-dione,
- 6-[p-(N,N-diéthylamino)phényl]quinoline-5,8-dione,
- 6-[p-(N,N-diméthylamino)phényl]quinoline-5,8-dione,
- 7-[p-(N,N-diméthylamino)phényl]quinoline-5,8-dione,
- 6-[4-(N,N-diéthylamino)-2-isopropénylphényl]quinoline-5,8-dione,
- 6-[4-(N,N-diéthylamino)-2-(N-acétylamino)phényl]quinoline-5,8-dione,
- 6-[4-(N,N-diméthylamino)-2-isopropénylphényl]quinoline-5,8-dione,
- 6-[4-(N,N-di-n-butylamino)-2-hydroxyphényl]quinoline-5,8-dione,
- 6-[p-(N-n-butylamino)phényl]quinoline-5,8-dione,
- 6-[p-(N-méthylamino)phényl]quinoline-5,8-dione,
- 6-(1-méthyl-1H-pyrrol-2-yl)quinoline-5,8-diol,
- 7-(1-méthyl-1H-pyrrol-2-yl)quinoline-5,8-diol.

4. Utilisation des composés de formule (I) ou (II) tels que définis dans la revendication 1, en coloration directe de cheveux.

5. Utilisation des composés de formule (I) ou (II) tels que définis dans la revendication 1, à titre de colorants directs en coloration d'oxydation de cheveux.

6. Utilisation des composés de formule (II) tels que définis dans la revendication 1, en coloration progressive de cheveux.

7. Composés nouveaux répondant aux formules (I) et (II) selon la revendication 1, sous réserve que :
lorsque Q représente 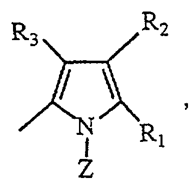 X=CH et T = H, alors Z est différent du groupe méthyle ;
lorsque Q représente 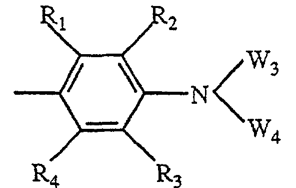 et l'un des substituants W₃ et W₄ représente un atome d'hydrogène, l'autre substituant ne représente pas un groupe méthyle ou n-butyle; et
lorsque Q représente 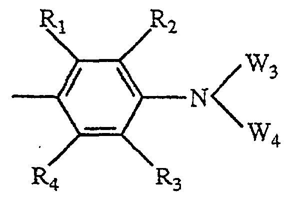 et les substituants W₃ et W₄ sont identiques, ils ne représentent pas un groupe méthyle, ni un groupe éthyle.

8. Composés nouveaux selon la revendication 7, caractérisés par le fait qu'ils sont choisis parmi les composés suivants :
- 6-(1-pentyl-1H-pyrrol-2-yl)quinoline-5,8-dione,
- 6-(1-benzyl-1H-pyrrol-2-yl)quinoline-5,8-dione,
- 4-(5,8-dioxo-5,8-dihydro-quinolin-6-yl)-2,5-diméthyl-1H-pyrrole-3-carboxylate de méthyle,
- N-[2-[2-(5,8-dioxo-5,8-dihydro-quinolin-6-yl)pyrrol-1-yl]-éthyl]acétamide,
- 6-(1-phényl-1H-pyrrol-2-yl)quinoline-5,8-dione,
- 3-[2-(5,8-dioxo-5,8-dihydro-quinolin-6-yl)pyrrol-1-yl]propionitrile.
- 6-chloro-7-(1-méthyl-1H-pyrrol-2-yl)quinoline-5,8-dione,
- 6-(1,2-diméthyl-1H-indol-3-yl)quinoline-5,8-dione,
- 6-(1-benzyl-1H-indol-3-yl)quinoline-5,8-dione,
- 6-[[p-(N,N-(2-diacétyloxyéthyl)amino]phényl]quinoline-5,8-dione,
- 6-(2-phényl-1H-indol-3-yl)quinoline-5,8-dione,
- 6-(1-méthyl-1H-indol-3-yl)quinoline-5,8-dione,
- 6-(2-méthyl-1H-indol-3-yl)quinoline-5,8-dione,
- 6-(1-méthyl-1H-pyrrol-2-yl)quinoxaline-5,8-dione.

9. Composition tinctoriale pour matières kératiniques, caractérisée en ce qu'elle comprend, dans un milieu approprié pour la teinture, une quantité efficace d'au moins un des composés de formule (I) ou (II) définis à l'une quelconque des revendications 1 à 8.

10. Composition cosmétique destinée à la teinture des matières kératiniques humaines, caractérisée en ce qu'elle comprend, dans un milieu cosmétiquement acceptable, une quantité efficace d'au moins un des composés de formule (I) ou (II) définis à l'une quelconque des revendications 1 à 8.

11. Procédé de teinture des fibres kératiniques humaines et notamment des cheveux, par coloration directe, caractérisé en que l'on applique une composition tinctoriale renfermant un composé de formule (I) ou (II) tel que défini dans l'une quelconque des revendications 1 à 8, sur les fibres kératiniques et qu'après un temps de pose suffisant pour développer la coloration désirée, on rince, on lave éventuellement, on rince à nouveau et on sèche ces fibres.

12. Procédé selon la revendication 11, caractérisé en ce que l'on applique une composition tinctoriale renfermant un composé de formule (I) ou (II) tel que défini dans l'une quelconque des revendications 1 à 8, sur les fibres kératiniques pendant un temps de pose suffisant pour développer la coloration désirée sans rinçage final.

13. Procédé de teinture progressive des fibres kératiniques humaines et notamment des cheveux, caractérisé en que l'on applique une composition tinctoriale renfermant un composé de formule (II) tel que défini dans l'une quelconque des revendications 1 à 8, sur les fibres kératiniques, que l'on expose les fibres à l'oxygène de l'air, on rince et on sèche les fibres et que l'on renouvelle l'application jusqu'à la coloration souhaitée.

## Patentansprüche

1. Verwendung der Verbindungen der allgemeinen Formeln (I) und (II) 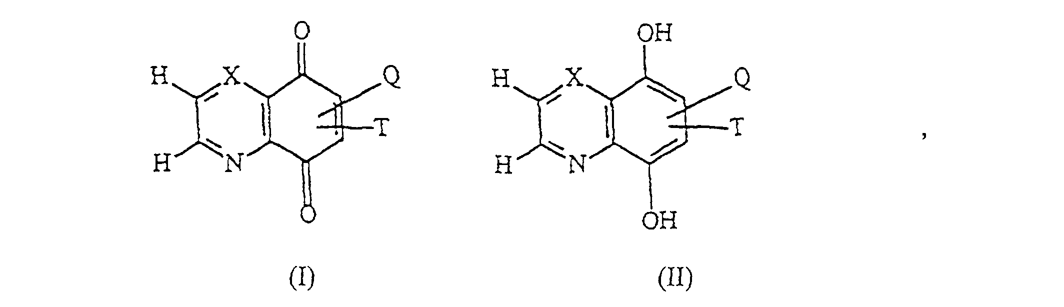 zur Färbung von Keratinmaterialien und insbesondere von menschlichen Keratinfasern, in denen bedeuten:
Q die Gruppen 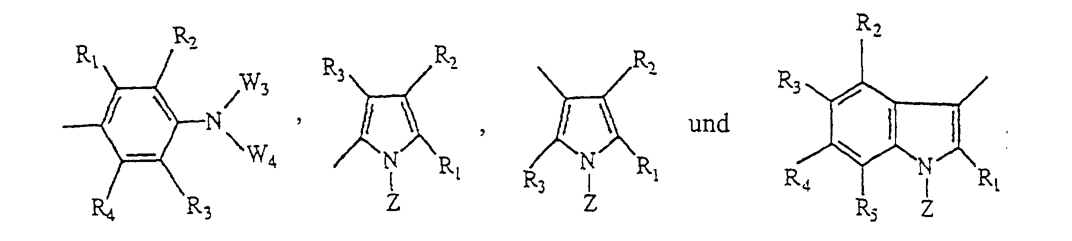
X die Gruppe CH oder ein Stickstoffatom,
T ein Wasserstoffatom, ein Halogenatom oder eine Hydroxygruppe,
R₁, R₂, R₃, R₄ und R₅, die gleich oder verschieden sind, Gruppen, die ausgewählt sind unter Wasserstoff, C₁-C₄-Alkoxygruppen, den Aminogruppen -N(W₁)(W₂), den Gruppen -NH-CO-W₁, den Gruppen -O-CO-W₁, der Hydroxygruppe, der Carbonsäuregruppe oder ihren Derivaten, wie den Estern, Amiden, anorganischen Salze oder Salzen organischer Amine, Halogenatomen, geradkettigen oder verzweigten C₁-C₄-Alkylgruppen, die gegebenenfalls eine Nitrilgruppe, eine Carbonsäuregruppe oder ihre Derivate, wie die Ester, Amide, anorganischen Salze oder Salze organischer Amine, eine Hydroxygruppe, eine Gruppe wie 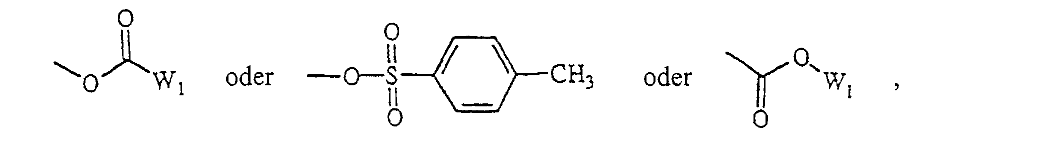 eine Ethergruppe -OSi(W₁)₃, eine Aminogruppe 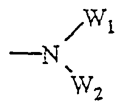 oder eine Amidogruppe 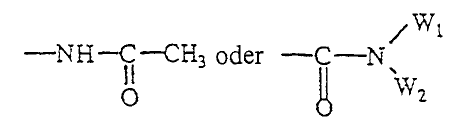 als Substituenten aufweisen,
Z ein Wasserstoffatom, eine Benzylgruppe, eine Phenylgruppe, die gegebenenfalls eine Hydroxygruppe, eine Gruppe -O-CO-W₁, eine Aminogruppe -N(W₁)(W₂), eine Gruppe -NH-CO-W₁ oder eine Nitrilgruppe als Substituenten aufweist, eine geradkettige oder verzweigte C₁-C₈-Alkylgruppe, die gegebenenfalls eine Nitrilgruppe, eine Carbonsäuregruppe oder ihre Derivate, wie die Ester, Amide, anorganischen Salze oder Salze organischer Amine, eine Hydroxygruppe, einer Gruppe wie 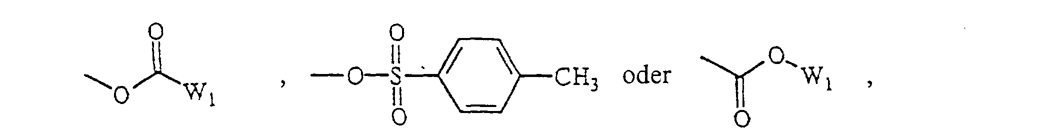 eine Ethergruppe -OSi(W₁)₃, eine Aminogruppe 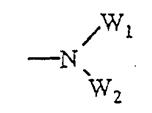 oder eine Amidogruppe 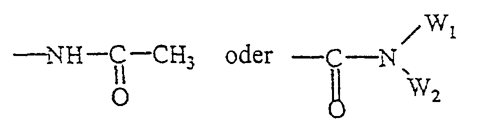 als Substituenten aufweist,
W₁ und W₂, die gleich oder verschieden sind, Gruppen, die unter Wasserstoff, geradkettigem oder verzweigtem C₁-C₄-Alkyl ausgewählt sind,
W₃ und W₄, die gleich oder verschieden sind, Gruppen, die ausgewählt sind unter Wasserstoff und geradkettigen oder verzweigten C₁-C₄-Alkylgruppen, die gegebenenfalls eine oder mehrere Hydroxygruppen, eine oder mehrere Gruppen -CO-O-W₁ oder -O-CO-W₁ oder eine oder mehrere Aminogruppen -N(W₁)(W₂) als Substituenten aufweisen.

2. Verwendung nach Anspruch 1, dadurch gekennzeichnet, daß es sich bei den Keratinfasern um Haare handelt.

3. Verwendung nach einem der vorhergehenden Ansprüche, dadurch gekennzeichnet, daß die Verbindungen der Formel (I) oder (II) unter den folgenden Verbindungen ausgewählt sind:
- 6-(1-Pentyl-1H-pyrrol-2-yl)-chinolin-5,8-dion,
- 6-(1-Benzyl-1H-pyrrol-2-yl)-chinolin-5,8-dion,
- 4-(5,8-Dioxo-5,8-dihydrochinolin-6-yl)-2,5-dimethyl-1H-pyrrol-3-carbonsäure methylester,
- N-[2-[2-(5,8-Dioxo-5,8-dihydrochinolin-6-yl)-pyrrol-1-yl]-ethyl]-acetamid,
- 6-(1-Phenyl-1H-pyrrol-2-yl)-chinolin-5,8-dion,
- 3-[2-(5,8-Dioxo-5,8-dihydrochinolin-6-yl)-pyrro1-1-yl]-propionitril,
- 6-Chlor-7-(1-methyl-1H-pyrrol-2-yl)-chinolin-5,8-dion,
- 6-(1,2-Dimethyl-1H-indol-3-yl)-chinolin-5,8-dion,
- 6-(1-Benzyl-1H-indol-3-yl)-chinolin-5,8-dion,
- 6-(2-Phenyl-1H-indol-3-yl)-chinolin-5,8-dion,
- 6-(1-Methyl-1H-indol-3-yl)-chinolin-5,8-dion,
- 6-(2-Methyl-1H-indol-3-yl)-chinolin-5,8-dion,
- 6-[p-(N,N-(2-Diacetyloxyethyl)-amino)-phenyl]-chinolin-5,8-dion,
- 6-(1-Methyl-1H-pyrrol-2-yl)-chinoxalin-5,8-dion,
- 6-(1-Methyl-1H-pyrrol-2-yl)-chinolin-5,8-dion,
- 7-(1-Methyl-1H-pyrrol-2-yl)-chinolin-5,8-dion,
- 6-[p-(N,N-Diethylamino)-phenyl]-chinolin-5,8-dion,
- 6-[p-(N,N-Dimethylamino)-phenyl]-chinolin-5,8-dion,
- 7-[p-(N,N-Dimethylamino)-phenyl]-chinolin-5,8-dion,
- 6-[4-(N,N-Diethylamino)-3-isopropenylphenyl]-chinolin-5,8-dion,
- 6-[4-(N,N-Diethylamino)-3-(N-acetylamino)-phenyl]-chinolin-5,8-dion,
- 6-[4-(N,N-Dimethylamino)-2-isopropenylphenyl]-chinolin-5,8-dion,
- 6-[4-(N,N-(Di-n-butylamino)-2-hydroxyphenyl]-chinolin-5,8-dion,
- 6-[p-(N-n-Butylamino)-phenyl]-chinolin-5,8-dion,
- 6-[p-(N-Methylamino)-phenyl]-chinolin-5,8-dion,
- 6-(1-Methyl-1H-pyrrol-2-yl)-chinolin-5,8-diol,
- 7-(1-Methyl-1H-pyrrol-2-yl)-chinolin-5,8-diol.

4. Verwendung der wie in Anspruch 1 definierten Verbindungen der Formel (I) oder (II) zur direktziehenden Färbung der Haare.

5. Verwendung der wie in Anspruch 1 definierten Verbindungen der Formel (I) oder (II) als direktziehende Farbstoffe bei der oxidativen Färbung der Haare.

6. Verwendung der wie in Anspruch 1 definierten Verbindungen der Formel (II) bei der progressiven Färbung der Haare.

7. Neue Verbindungen der Formeln (I) und (II) gemäß Anspruch 1 mit der Maßgabe daß X die Gruppe CH, T ein Wasserstoffatom und Z eine Gruppe, die von Methyl verschieden ist, bedeutet, wenn
Q die Gruppe 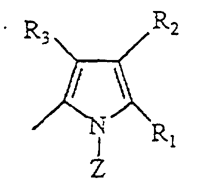 darstellt;
daß der eine der beiden Substituenten W₃ und W₄ weder Methyl noch n-Butyl darstellt,
wenn Q die Gruppe 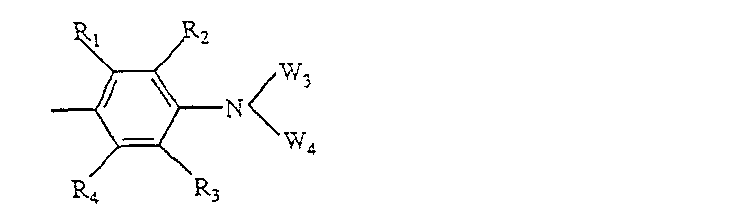
darstellt und der andere der beiden Substituenten W₃ und W₄ ein Wasserstoffatom bedeutet; und
daß die beiden Substituenten W₃ und W₄ weder Methyl noch Ethyl bedeuten, wenn Q die Gruppe 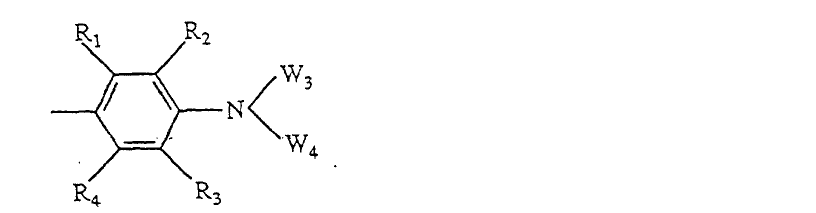 darstellt und die Substituenten W₃ und W₄ identisch sind.

8. Neue Verbindungen nach Anspruch 7, dadurch gekennzeichnet, daß sie unter den folgenden Verbindungen ausgewählt sind:
- 6-(1-Pentyl-1H-pyrrol-2-yl)-chinolin-5,8-dion,
- 6-(1-Benzyl-1H-pyrrol-2-yl)-chinolin-5,8-dion,
- 4-(5,8-Dioxo-5,8-dihydrochinolin-6-yl)-2,5-dimethyl-1H-pyrrol-3-carbonsäure methylester,
- N-[2-[2-(5,8-Dioxo-5,8-dihydrochinolin-6-yl)-pyrrol-1-yl]-ethyl]-acetamid,
- 6-(1-Phenyl-1H-pyrrol-2-yl)-chinolin-5,8-dion,
- 3-[2-(5,8-Dioxo-5,8-dihydrochinolin-6-yl)-pyrrol-1-yl]-propionitril,
- 6-Chlor-7-(1-methyl-1H-pyrrol-2-yl)-chinolin-5,8-dion,
- 6-(1,2-Dimethyl-1H-indol-3-yl)-chinolin-5,8-dion,
- 6-(1-Benzyl-1H-indol-3-yl)-chinolin-5,8-dion,
- 6-[[p-(N,N-(2-Diacetyloxtyethyl)-amino]-phenyl]-chinolin-5,8-dion,
- 6-(2-Phenyl-1H-indol-3-yl)-chinolin-5,8-dion,
- 6-(1-Methyl-1H-indol-3-yl)-chinolin-5,8-dion,
- 6-(2-Methyl-1H-indol-3-yl)-chinolin-5,8-dion,
- 6-(1-Methyl-1H-pyrrol-2-yl)-chinoxalin-5,8-dion,

9. Färbemittelzusammensetzung für Keratinmaterialien, dadurch gekennzeichnet, daß sie in einem für die Färbung geeigneten Medium eine wirksame Menge mindestens einer der in einem der Ansprüche 1 bis 8 definierten Verbindungen der Formeln (I) und (II) enthält.

10. Kosmetische Zusammensetzung, die für die Färbung menschlicher Keratinmaterialien vorgesehen ist, dadurch gekennzeichnet, daß sie in einem kosmetisch akzeptablen Medium eine wirksame Menge mindestens eine der in einem der Ansprüche 1 bis 8 definierten Verbindungen der Formel (I) oder (II) enthält.

11. Verfahren zur Färbung menschlicher Keratinfasern und insbesondere der Haare durch direktziehende Färbung, dadurch gekennzeichnet, daß eine Färbemittelzusammensetzung, die eine wie in einem der Ansprüche 1 bis 8 definierte Verbindung der Formel (I) oder (II) enthält, auf die Keratinfasern aufgetragen wird und daß nach einer Einwirkungszeit, die ausreichend ist, um die gewünschte Farbe zu entwickeln, die Fasern ausgespült, gegebenenfalls gewaschen, erneut ausgespült und dann getrocknet werden.

12. Verfahren nach Anspruch 11, dadurch gekennzeichnet, daß eine Färbemittelzusammensetzung, die eine wie in einem der Ansprüche 1 bis 8 definierte Verbindung der Formel (I) oder (II) enthält, nach dem Auftragen während einer Einwirkungszeit, die ausreichend ist, um die gewünschte Farbe zu entwickeln, auf den Keratinfasern belassen wird, ohne daß die Keratinfasern abschließend ausgespült werden.

13. Verfahren zur progressiven Färbung menschlicher Keratinfasern und insbesondere der Haare, dadurch gekennzeichnet, daß eine Färbemittelzusammensetzung, die eine wie in einem der Ansprüche 1 bis 8 definierte Verbindung der Formel (II) enthält, auf die Keratinfasern aufgetragen wird, daß die Fasern dem Luftsauerstoff ausgesetzt und dann ausgespült und getrocknet werden und daß dieser Vorgang wiederholt wird, bis die gewünschte Farbe erhalten wird.

## Claims

1. Use for the dyeing of keratinous matter, in particular of human keratinous fibres, of compounds of general formulae (I) and (II): 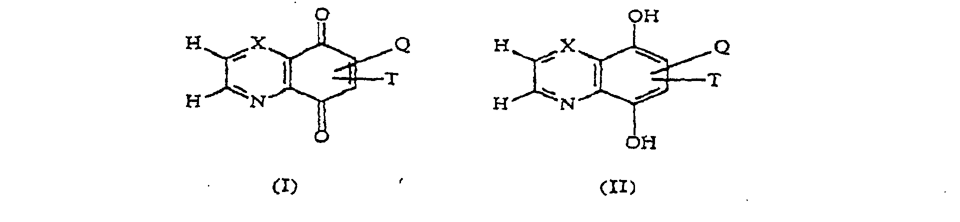 in which:
Q represents the groups 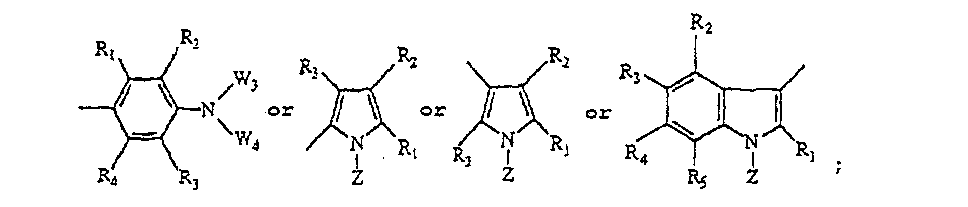
X represents the group CH or a nitrogen atom;
T represents a hydrogen atom, a halogen atom or a hydroxyl group;
R₁, R₂, R₃, R₄ and R₅, which are identical or different, are selected from a hydrogen atom, a C₁-C₄ alkoxy group, an amino group -N(W₁)(W₂), a group -NH-CO-W₁, a group -O-CO-W₁, a hydroxyl group, a carboxylic acid group or its derivatives such as esters, amides or mineral salts or organic amine salts, a halogen atom, a linear or branched C₁-C₄ alkyl group optionally substituted by a nitrile group, by a carboxylic acid group or its derivatives such as esters, amides or mineral salts or organic amine salts, by a hydroxyl group, by a group such as: 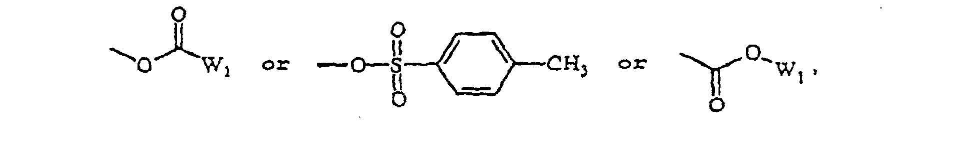 by an ether group -OSi(W₁)₃, by an amino group 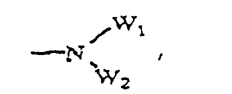 or by an amido group 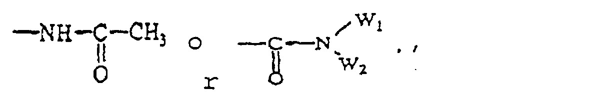
Z represents a hydrogen atom, a benzyl group, a phenyl group optionally substituted by a hydroxyl group, a group -O-CO-W₁, an amino group -M(W₁)(W₂), a group -NH-CO-W₁ or a nitrile group; a linear or branched C₁-C₈ alkyl group optionally substituted by a nitrile group, by a carboxylic acid group or its derivatives such as esters, amides or mineral salts or organic amine salts, by a hydroxyl group, by a group such as: 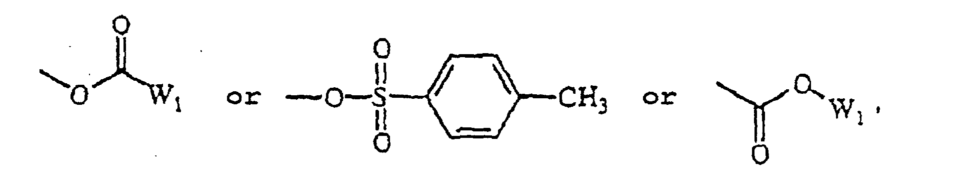 by an ether group -OSi(W₁)₃, by an amino group 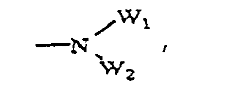 or by an amido group 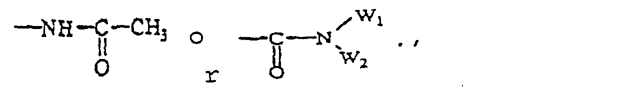
W₁ and W₂, which are identical or different, are selected from a hydrogen atom and a linear or branched C₁-C₄ alkyl group,
W₃ and W₄, which are identical or different, are selected from a hydrogen atom and a linear or branched C₁-C₄ alkyl group optionally substituted by one or more hydroxyl groups, by one or more groups -CO-O-W₁ or -O-CO-W₁, or by one or more amino groups -N(W₁) (W₂).

2. Use according to Claim 1, characterized in that it is applied to hair.

3. Use according to either of the preceding claims, characterized in that the compounds of formula (I) or (II) are selected from the following compounds:
- 6-(1-pentyl-1H-pyrrol-2-yl)quinoline-5,8-dione,
- 6-(1-benzyl-1H-pyrrol-2-yl)quinoline-5,8-dione,
- methyl 4-(5,8-dioxo-5,8-dihydroquinolin-6-yl)-2,5-dimethyl-1H-pyrrole-3-carboxylate,
- N-[2-[2-(5,8-dioxo-5,8-dihydroquinolin-6-yl)pyrrol-1-yl]ethyl]acetamide,
- 6-(1-phenyl-1H-pyrrol-2-yl)quinoline-5,8-dione,
- 3-[2-(5,8-dioxo-5,8-dihydroquinolin-6-yl)pyrrol-1-yl]propionitrile,
- 6-chloro-7-(1-methyl-1H-pyrrol-2-yl)quinoline-5,8-dione,
- 6-(1,2-dimethyl-1H-indol-3-yl)quinoline-5,8-dione,
- 6-(1-benzyl-1H-indol-3-yl)quinoline-5,8-dione,
- 6-(2-phenyl-1H-indol-3-yl)quinoline-5,8-dione,
- 6-(1-methyl-1H-indol-3-yl)quinoline-5,8-dione,
- 6-(2-methyl-1H-indol-3-yl)quinoline-5,8-dione,
- 6-[p-(N,N-(2-diacetyloxyethyl)amino)phenyl]-quinoline-5,8-dione,
- 6-(1-methyl-1H-pyrrol-2-yl)quinoxaline-5,8-dione,
- 6-(1-methyl-1H-pyrrol-2-yl)quinoline-5,8-dione,
- 7-(1-methyl-1H-pyrrol-2-yl)quinoline-5,8-dione,
- 6-[p-(N,N-diethylamino)phenyl]quinoline-5,8-dione,
- 6-[p-(N,N-dimethylamino)phenyl]quinoline-5,8-dione,
- 7-[p-(N,N-dimethylamino)phenyl]quinoline-5,8-dione,
- 6-[4-(N,N-diethylamino)-2-isopropenylphenyl]-quinoline-5,8-dione,
- 6-[4-(N,N-diethylamino)-2-(N-acetylamino)phenyl]-quinoline-5,8-dione,
- 6-[4-(N,N-dimethylamino)-2-isopropenylphenyl]-quinoline-5,8-dione,
- 6-[4-(N,N-di-n-butylamino)-2-hydroxyphenyl]-quinoline-5,8-dione,
- 6-[p-(N-n-butylamino)phenyl]quinoline-5,8-dione,
- 6-[p-(N-methylamino)phenyl]quinoline-5,8-dione,
- 6-(1-methyl-1H-pyrrol-2-yl)quinoline-5,8-diol,
- 7-(1-methyl-1H-pyrrol-2-yl)quinoline-5,8-diol.

4. Use of compounds of formula (I) or (II) as defined in Claim 1 for the direct dyeing of hair.

5. Use of compounds of formula (I) or (II) as defined in Claim 1 as direct dyes in the oxidation dyeing of hair.

6. Use of compounds of formula (II) as defined in Claim 1 for the progressive dyeing of hair.

7. Novel compounds corresponding to the formulae (I) and (II) according to Claim 1, with the proviso that:
when Q represents 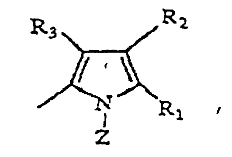 X = CH and T=H, then Z is other than the methyl group;
when Q represents 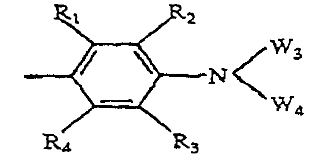 and one of the substituents W₃ and W₄ represents a hydrogen atom, the other substituent does not represent a methyl or n-butyl group; and
when Q represents 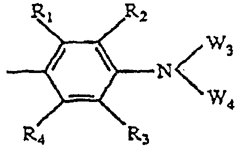 and the substituents W₃ and W₄ are identical, they do not represent either a methyl group or an ethyl group.

8. Novel compounds according to Claim 7, characterized in that they are selected from the following compounds:
- 6-(1-pentyl-1H-pyrrol-2-yl)quinoline-5,8-dione,
- 6-(1-benzyl-1H-pyrrol-2-yl)quinoline-5,8-dione,
- methyl 4-(5,8-dioxo-5,8-dihydroquinolin-6-yl)-2,5-dimethyl-1H-pyrrole-3-carboxylate,
- N-[2-[2-(5,8-dioxo-5,8-dihydroquinolin-6-yl)pyrrol-1-yl] ethyl] acetamide,
- 6-(1-phenyl-1H-pyrrol-2-yl)quinoline-5,8-dione,
- 3-[2-(5,8-dioxo-5,8-dihydroquinolin-6-yl)pyrrol-1-yl]propionitrile,
- 6-chloro-7-(1-methyl-1H-pyrrol-2-yl)quinoline-5,8-dione,
- 6-(1,2-dimethyl-1H-indol-3-yl)quinoline-5,8-dione,
- 6-(1-benzyl-1H-indol-3-yl)quinoline-5,8-dione,
- 6-[p-(N,N-(2-diacetyloxyethyl)amino)phenyl]-quinoline-5,8-dione,
- 6-(2-phenyl-1H-indol-3-yl)quinoline-5,8-dione,
- 6-(1-methyl-1H-indol-3-yl)quinoline-5,8-dione,
- 6-(2-methyl-1H-indol-3-yl)quinoline-5,8-dione,
- 6-(1-methyl-1H-pyrrol-2-yl)quinoxaline-5,8-dione.

9. Dyeing composition for keratinous matter, characterized in that it comprises, in a medium appropriate for dyeing, an effective amount of at least one of the compounds of formula (I) or (II) defined in any one of Claims 1 to 8.

10. Cosmetic composition intended for the dyeing of human keratinous matter, characterized in that it comprises, in a cosmetically acceptable medium, an effective amount of at least one of the compounds of formula (I) or (II) defined in any one of Claims 1 to 8.

11. Method of dyeing human keratinous fibres and, in particular, the hair by direct dyeing, characterized in that a dyeing composition comprising a compound of formula (I) or (II) as defined in any one of Claims 1 to 8 is applied to the keratinous fibres and in that, after a time sufficient to develop the desired colouration, the fibres are rinsed, optionally washed, rinsed again and dried.

12. Method according to Claim 11, characterized in that a dyeing composition comprising a compound of formula (I) or (II) as defined in any one of Claims 1 to 8 is applied to the keratinous fibres for a time sufficient to develop the desired colouration without final rinsing.

13. Method of progressive dyeing of human keratinous fibres and, in particular, the hair, characterized in that a dyeing composition comprising a compound of formula (II) as defined in any one of Claims 1 to 8 is applied to the keratinous fibres, and in that the fibres are exposed to atmospheric oxygen, and the fibres are rinsed and dried, and in that the application is repeated until the desired colouration is obtained.
